# EUROPÄISCHE PATENTSCHRIFT

(11) **EP 2 886 042 B1**
(45) Veröffentlichungstag und Bekanntmachung des Hinweises auf die Patenterteilung: **02.11.2022**
(21) Anmeldenummer: 13198856.0
(22) Anmeldetag: 20.12.2013
(51) Int. Cl.: A61B 3/14, A61B 3/00, A61F 9/013

(54) **ANORDNUNG UND VERFAHREN ZUM VERGLEICHEN EINER AUGENSTRUKTUR MIT EINER STRUKTURABBILDUNG**
ASSEMBLY AND METHOD FOR COMPARING AN EYE STRUCTURE WITH A STRUCTURE IMAGE
AGENCEMENT ET PROCÉDÉ DESTINÉS À LA COMPARAISON D'UNE STRUCTURE OCULAIRE AVEC UNE REPRÉSENTATION STRUCTURELLE

(43) Veröffentlichungstag der Anmeldung: 24.06.2015
(73) Patentinhaber: Phronema S.r.l., 70125 Bari (BA) (IT)
(72) Erfinder: Ehrhorn, Wiebke, 22880 Wedel (DE); Gerike, Joachim, 22880 Wedel (DE)
(74) Vertreter: Bernotti, Andrea

(56) Entgegenhaltungen:
- WO-A2-2011/116306
- MICHAEL PIRCHER ET AL: "Polarization sensitive optical coherence tomography in the human eye", PROGRESS IN RETINAL AND EYE RESEARCH, Bd. 30, Nr. 6, 1. November 2011 (2011-11-01), Seiten 431-451, XP028318331, ISSN: 1350-9462, DOI: 10.1016/J.PRETEYERES.2011.06.003 [gefunden am 2011-06-26]
- GARY P. MISSON: "Circular polarization biomicroscopy: a method for determining human corneal stromal lamellar organization in vivo", OPHTHALMIC AND PHYSIOLOGICAL OPTICS, Bd. 27, Nr. 3, 1. Mai 2007 (2007-05-01), Seiten 256-264, XP055119909, ISSN: 0275-5408, DOI: 10.1111/j.1475-1313.2007.00482.x

## Beschreibung

Die Erfindung betrifft eine Anordnung und ein Verfahren zum Vergleichen einer Augenstruktur mit einer Strukturabbildung.

In der Augenmedizin gibt es verschiedene Anlässe, eine Augenstruktur mit einer Strukturabbildung zu vergleichen. Der Vergleich kann beispielsweise dazu dienen, ein Auge zu identifizieren, um sicherzustellen, dass eine Behandlung am richtigen Auge vorgenommen wird. Ebenso ist es in einigen Fällen von Interesse, die Position oder Ausrichtung eines Auges zu ermitteln. Auch eine Veränderung von Strukturen des Auges oder eine Abweichung von der Norm lässt sich auf diese Weise ermitteln.

Bislang orientieren Augenärzte sich an sichtbaren Strukturen des Auges um einen entsprechenden Vergleich durchzuführen. Dies können beispielsweise kleine Adern oder sonstige Markierungen auf der Sklera sein oder bestimmte Merkmale der Iris. Die Merkmale der Sklera verändern sich bei der Manipulation am Auge während der Operation sowie im Laufe der Zeit, so dass ein aussagekräftiger Vergleich nur möglich ist, wenn die Strukturabbildung kurz vorher aufgenommen wurde. Die Merkmale der Iris sind nur schwierig zu identifizieren, wenn die Pupille mit Medikamenten aufgeweitet wurde.

Die Patentanmeldung WO 2011/116306 A2 offenbart ein Monitoring der Vernetzung von Kollagen der Kornea.

Der Erfindung liegt die Aufgabe zu Grunde, ein Verfahren und eine Anordnung vorzustellen, die einen zuverlässigen Vergleich einer Augenstruktur mit einer Strukturabbildung ermöglichen. Die Aufgabe wird gelöst mit den Merkmalen der unabhängigen Ansprüche. Vorteilhafte Ausführungsformen sind in den Unteransprüchen angegeben.

Die erfindungsgemäße Anordnung umfasst einen Speicher, in dem die Strukturabbildung gespeichert werden kann. Eine Lichtquelle, die mit einem ersten linearen Polarisator versehen ist, ist dazu ausgelegt, linear polarisiertes Messlicht auf das Auge zu richten. Ein Bildsensor ist dazu ausgelegt, von dem Auge zurückgeworfene Anteile des Messlichts zu ermitteln. Ein zweiter linearer Polarisator ist zwischen dem Auge und dem Bildsensor angeordnet. Die Polarisationsrichtung des ersten Polarisators schließt mit der Polarisationsrichtung des zweiten Polarisators einen Winkel zwischen 75° und 105°, vorzugsweise einen Winkel zwischen 85° und 95°, weiter vorzugsweise einen Winkel von 90° ein. Schließlich umfasst die Anordnung einen Komparator, dazu ausgelegt ist, ein durch den Bildsensor gewonnenes Bild mit der Strukturabbildung zu vergleichen. Das gewonnene Bild und die Strukturabbildung enthalten Informationen über die Anordnung der Kollagen-Strukturen in der Cornea.

Mit der Erfindung werden Augenstrukturen für den Vergleich mit der Strukturabbildung nutzbar gemacht, die im Allgemeinen nicht sichtbar sind. Es handelt sich um die Kollagen-Strukturen im Stroma der Cornea (Hornhaut) des Auges. Da die Cornea durchsichtig ist, können die Kollagen-Strukturen mit normalen optischen Untersuchungen regelmäßig nicht identifiziert werden. Bei der erfindungsgemäßen Anordnung wird das normal reflektierte Messlicht zum größten Teil blockiert. Trifft linear polarisiertes Licht auf einen Polarisator, dessen Polarisationsrichtung im Wesentlichen rechtwinklig zu dem Licht ausgerichtet ist, kann das Licht nicht oder nur zu sehr geringen Anteilen durch den Polarisator hindurchtreten. Da sich die Polarisationsrichtung des Lichts durch eine Reflexion im Allgemeinen nicht ändert, wird der reflektierte Anteil des Messlichts durch den Polarisator blockiert. Schließen die beiden Polarisationsrichtungen einen Winkel von exakt 90° ein, so wird das direkt reflektierte Licht theoretisch vollständig blockiert. Ist der Winkel nur annähernd 90°, können noch geringe Anteile des direkt reflektierten Lichts hindurchtreten.

Mit der Erfindung wird ausgenutzt, dass die Kollagen-Strukturen der Cornea eine optische Aktivität aufweisen. Dies bedeutet, dass die Polarisationsrichtung des Messlichts durch die Kollagen-Strukturen verändert wird. Messlicht mit veränderter Polarisationsrichtung tritt aber durch den zweiten Polarisator hindurch, so dass das Licht auf den hinter dem zweiten Polarisator angeordneten Bildsensor trifft. Der Bildsensor, der beispielsweise als CCD-Sensor ausgebildet sein kann, kann ein Bild des durch den zweiten Polarisator hindurchgetretenen Messlichts aufnehmen. Das Bild enthält Informationen über die Lage der Kollagen-Strukturen. Durch den Vergleich mit einer zuvor bereitgestellten Strukturabbildung der Kollagen-Strukturen desselben Auges, eines anderen Auges oder einer Musterstruktur kann der Augenarzt relevante Informationen gewinnen. Diese Informationen können sich beispielsweise auf eine Identifizierung des Auges, auf die Ausrichtung bzw. die Position des Auges beziehen oder auf eine Veränderung des Auges beziehen.

Die beiden Polarisatoren sollten so zueinander ausgerichtet sein, dass das von den Kollagen-Strukturen reflektierte Licht noch von dem direkt reflektierten Licht unterschieden werden kann. Ist das direkt reflektierte Licht zu hell, können die Polarisatoren so zueinander ausgerichtet werden, dass weniger direkt reflektiertes Licht hindurchtreten kann. Am besten lassen sich die Kollagen-Strukturen in aller Regel erkennen, wenn die beiden Polarisationsrichtungen einen Winkel von 90° zwischen sich einschließen.

Mit der erfindungsgemäßen Anordnung lässt sich eine hohe Empfindlichkeit realisieren, so dass bereits geringe Lichtmengen, die aufgrund ihrer geänderten Polarisationsrichtung durch den zweiten Polarisator hindurchtreten, detektiert werden können. Dies ist auch erforderlich, weil bei einer bestimmten Ausrichtung der Polarisatoren nur ein Teil des von den Kollagen-Strukturen zurückgeworfenen Lichts detektiert werden kann. Je nach Ausrichtung der Polarisatoren sind es folglich nur geringe Lichtmengen, die von den Kollagen-Strukturen durch den zweiten Polarisator hindurchtreten können.

Wird die Ausrichtung der Polarisation des ersten und zweiten Polarisators geändert (wobei der Winkel zwischen der Polarisationsrichtung des ersten Polarisators und der Polarisationsrichtung des zweiten Polarisators vorzugsweise beibehalten wird), kann ein anderer Teil des von den Kollagen-Strukturen ausgehenden Messlichts durch den zweiten Polarisator hindurchtreten. Es lassen sich also zusätzliche Informationen über die Lage und Anordnung der Kollagen-Strukturen gewinnen, wenn man unter verschiedenen Ausrichtungen der Polarisatoren mit dem Bildsensor eine Aufnahme macht. Um Aufnahmen unter verschiedenen Ausrichtungen der Polarisatoren zu ermöglichen, können der erste Polarisator und der zweite Polarisator drehbar gelagert sein. Die Drehachse kann parallel zur Ausbreitungsrichtung des jeweils hindurchtretenden Messlichts sein.

Um sicherzustellen, das trotz der Drehung der Polarisatoren der Winkel zwischen den beiden Polarisationsrichtungen erhalten bleibt, können die beiden Polarisatoren miteinander gekoppelt sein. Es kann sich um eine mechanische Kopplung handeln. Möglich ist auch eine indirekte Kopplung über Sensoren und Aktoren, durch die sichergestellt wird, dass die Polarisatoren um den gleichen Drehwinkel gedreht werden.

In einer bevorzugten Ausführungsform sind der erste Polarisator und der zweite Polarisator konzentrisch zueinander angeordnet. Dies bietet die Möglichkeit, die beiden Polarisatoren zu einer Einheit zu verbinden und gemeinsam zu drehen. Der erste Polarisator kann außen angeordnet sein und der zweite Polarisator innen. Möglich ist auch die umgekehrte Anordnung.

Wird bei unterschiedlichen Ausrichtungen der Polarisation jeweils eine Aufnahme mit dem Bildsensor gemacht, so gewinnt man eine Mehrzahl von Einzelbildern des durch den zweiten Polarisator hindurchgetretenen Messlichts. Es ist möglich, ein Einzelbild mit der gespeicherten Strukturabbildung zu vergleichen. In einer vorteilhaften Ausführungsform wird eine Mehrzahl von Einzelbildern zu einem Gesamtbild überlagert und das Gesamtbild mit der gespeicherten Strukturabbildung verglichen. Das Gesamtbild hat einen deutlich höheren Informationsgehalt hinsichtlich der Kollagen-Strukturen, so dass der Vergleich mit dem Gesamtbild aussagekräftiger ist. Die Anordnung kann ein Bildmodul umfassen, das dazu ausgelegt ist, eine Mehrzahl von Einzelbildern des Bildsensors zu einem Gesamtbild zu überlagern. Der Komparator kann dazu ausgelegt sein, einen Vergleich zwischen dem Gesamtbild und der Strukturabbildung durchzuführen.

Überlagern bedeutet, die Informationen aus den Einzelbildern so zusammenzuführen, dass in dem Gesamtbild die Informationen der Einzelbilder addiert sind. Der Begriff überlagern ist nicht einschränkend im Hinblick auf eine bestimmte Art der Generierung oder Berechnung des Gesamtbilds zu verstehen.

Das Verfahren kann von Hand durchgeführt werden, indem die Ausrichtung der Polarisation jeweils manuell eingestellt wird und anschließend der Bildsensor ausgelöst wird. Bevorzugt ist eine automatische Durchführung des Verfahrens, bei dem eine Steuereinheit die Ausrichtung der Polarisation einstellt und den Bildsensor auslöst. Die Anordnung kann geeignete Aktoren für die Polarisatoren umfassen, über die die Steuereinheit die Ausrichtung der Polarisation einstellen kann.

Dreht man die Ausrichtung der Polarisation um 180°, ergibt sich für den Bildsensor das identische Bild wie bei 0°. Vorzugsweise wird eine Mehrzahl von Bildern aufgenommen, bei denen die Ausrichtung der Polarisation zwischen 0° und 180° liegen. Wird mit einer Mehrzahl von Aufnahmen ein Winkelbereich der Polarisation abgedeckt, so schließen benachbarte Ausrichtungen vorzugsweise gleiche Winkel miteinander ein. Beispielsweise kann der Winkelbereich von 0° bis 90° mit neunzig 1°-Schritten abgedeckt werden. Der Winkelbereich von 0° bis 180° kann mit sechzig 3°-Schritten abgedeckt werden. Das Gesamtbild kann aus der zur Verfügung stehenden Mehrzahl von Einzelbildern überlagert werden.

Der Vergleich, den der Komparator durchführt, kann darin bestehen, dass das durch den Bildsensor gewonnene Bild der Strukturabbildung gegenübergestellt wird, so dass eine Person den Grad an Übereinstimmung bzw. Abweichung einschätzen kann. In einer bevorzugten Ausführungsform ist der Komparator dazu ausgelegt, die Abweichung zwischen dem Bild und der Strukturabbildung automatisch auszuwerten. Es kann zu diesem Zweck ein automatischer Bildvergleich durchgeführt werden, aus dem sich ein Kennwert ergibt, der die Abweichung repräsentiert. Für den Vergleich ist es von Vorteil, eine vorherige Skalierung und/oder Anpassung des Bilds vorzunehmen. Dies hat das Ziel, einen möglichst hohen Grad an Übereinstimmung zwischen dem Bild und der Strukturabbildung zu erhalten. Die Anpassung des Bilds kann sich beispielsweise auf die Position, Ausrichtung, Kontrast, Schärfe und/oder Helligkeit beziehen.

Die Strukturabbildung ist in einer Ausführungsform eine Abbildung desselben Auges, die zu einem anderen Zeitpunkt aufgenommen wurde. Der Vergleich kann in diesem Fall dazu dienen, das Auge zu identifizieren oder eine Veränderung des Auges mit der Zeit zu ermitteln. Möglich ist auch, dass die Strukturabbildung von einem anderen Auge stammt oder eine Musterstruktur eines Auges wiedergibt. Ein Vergleich mit einem anderen Auge oder einer Musterstruktur kann dazu dienen, eine Abweichung des Auges von der Norm zu identifizieren. Beispielsweise kann ein Keratokonus oder ein Astigmatismus auf diese Weise erkannt werden.

Die Abweichung zwischen dem Bild von dem Bildsensor und der Strukturabbildung kann unterschiedlicher Art sein. Beispielsweise können das Bild und die Strukturabbildung relativ zueinander verschoben sein. Der Kennwert kann in diesem Fall eine Richtung und einen Abstand angeben, um das Bild und die Strukturabbildung zur Überdeckung zu bringen. Dies kann beispielsweise hilfreich sein, wenn anhand des mit dem Bildsensor gewonnenen Bilds ein Instrument in eine bestimmte Position geführt werden soll. Die Abweichung kann auch darin bestehen, dass das Bild relativ zu der Strukturabbildung gedreht ist. In diesem Fall kann der Kennwert einen Drehwinkel angeben, durch den das Bild und die Strukturabbildung zur Überdeckung gebracht werden können. Auch eine Kombination aus Verschiebung und Verdrehung ist möglich.

In einem anderen Fall kann die Abweichung zwischen dem Bild vom Bildsensor und der Strukturabbildung derart sein, dass sich weder durch Verdrehen noch durch Verschieben eine Überdeckung erzielen lässt. Passen die Strukturen gar nicht zusammen, stammt die Strukturabbildung möglicherweise von einem anderen Auge als das Bild vom Bildsensor. Diese Funktion kann genutzt werden, um fehlerhafte Eingriffe zu verhindern. Möglich ist auch, dass das Bild und die Strukturabbildung zwar vom gleichen Auge stammen, die Anordnung oder Ausrichtung der Kollagen-Strukturen sich aber in der Zwischenzeit verändert hat. Wird das Bild mit einer Musterstruktur verglichen, so kann eine Abweichung festgestellt werden. Solche Feststellungen können Anlass für weitergehende Untersuchungen des Auges sein.

Insbesondere um fehlerhafte Eingriffe zu verhindern, kann der Komparator dazu ausgelegt sein, ein Signal auszugeben, wenn die Abweichung zwischen dem Bild vom Bildsensor und der Strukturabbildung größer ist als ein vorgegebener Schwellwert. Der Schwellwert kann sich auf die Position und/oder die Ausrichtung des Bilds relativ zu der Strukturabbildung beziehen. In einer bevorzugten Ausführungsform bezieht sich der Schwellwert im Sinne einer Ähnlichkeitsanalyse auf die abgebildete Struktur. Vorzugsweise werden die Bilder vor der Ähnlichkeitsanalyse in die bestmögliche Übereinstimmung hinsichtlich Position und Ausrichtung gebracht.

Durch den ersten Polarisator wird sichergestellt, dass nur linear polarisiertes Messlicht auf das Auge auftrifft. Stammt das Messlicht von einer Lichtquelle, die linear polarisiertes Licht abgibt, so ist der Polarisator von der Lichtquelle umfasst. Gibt die Lichtquelle unpolarisiertes Licht ab, so kann der Polarisator als Polarisationsfilter ausgebildet sein, der zwischen der Lichtquelle und dem Auge angeordnet ist. Der zweite Polarisator ist als Polarisationsfilter ausgebildet. Wenn ein Bild mit dem Bildsensor aufgenommen wird, schließen die Polarisationsrichtung des ersten Polarisators und die Polarisationsrichtung des zweiten Polarisators einen rechten Winkel miteinander ein.

Die Lichtquelle kann sichtbares Licht ausstrahlen. Möglich ist auch eine Verwendung von Licht außerhalb des sichtbaren Bereichs oder eine Kombination von Wellenlängen innerhalb und außerhalb des sichtbaren Bereichs. Der nicht-sichtbare Bereich kann UV-Licht und/oder IR-Licht umfassen. Damit genügend Licht bei dem Bildsensor ankommt, ist es von Vorteil, eine Lichtquelle mit großer Helligkeit zu verwenden. Die Lichtquelle kann beispielsweise eine LED oder eine Anordnung mehrerer LEDs sein. Um das Messlicht von Umgebungslicht unterscheiden zu können, kann das Messlicht über der Zeit moduliert werden. Nimmt der Sensor Licht mit der entsprechenden Modulation auf, so handelt es sich um reflektiertes Messlicht und nicht um Umgebungslicht. Alternativ oder zusätzlich dazu kann im Strahlengang des Messlichts ein spektraler Filter angeordnet sein, um das Messlicht vom Umgebungslicht unterscheiden zu können.

Bei der Strukturabbildung, die für den Vergleich verwendet wird, kann es sich um eine Abbildung handeln, die zu einem früheren Zeitpunkt von demselben Auge erzeugt wurde. Es kann sich auch eine Abbildung eines anderen Auges oder um eine Musterstruktur handeln. Die Strukturabbildung enthält eine Information über die Anordnung der Kollagen-Strukturen in der Cornea. Die Strukturabbildung kann unter Ausnutzung der optischen Aktivität der Kollagen-Strukturen oder auf andere Weise gewonnen worden sein.

Die Erfindung ist von besonderem Nutzen, wenn die Anordnung ein weiteres Gerät umfasst, mit dem zusätzliche Informationen gewonnen werden können oder zusätzliche Schritte ermöglicht werden. Beispielsweise kann die Anordnung ein Operationsmikroskop und/oder ein Spaltlampenmikroskop für eine parallele optische Beobachtung des Auges umfassen. Das Mikroskop ist so angeordnet, dass dasselbe Auge beobachtet werden kann, auf dass das Messlicht gerichtet wird. Die optische Beobachtung kann gleichzeitig erfolgen wie die erfindungsgemäße Ermittlung der Kollagen-Strukturen. Möglich ist auch, diese Schritte nacheinander vorzunehmen. Die Anordnung kann eine integrierte Gestaltung aufweisen, was bedeutet, dass das Messlicht auf seinem Weg von der Lichtquelle zum Auge und/oder auf seinem Weg von dem Auge zu dem Bildsensor durch ein Hauptobjektiv des Operationsmikroskops bzw. des Spaltlampenmikroskops hindurchtritt. Es kann vorgesehen sein, dass ein durch den Bildsensor gewonnenes Bild dem optischen Bild im Mikroskop überlagert wird, so dass der Augenarzt beides gleichzeitig sehen kann.

Zusätzlich oder alternativ zu dem Operationsmikroskop kann die Anordnung ein Biometer umfassen, mit dem das Auge parallel vermessen werden kann. Vermessen bedeutet, dass die räumliche Form von Elementen des Auges und/oder die Anordnung von Elementen des Auges relativ zueinander ermittelt wird. Beispielsweise kann die Krümmung der Cornea ermittelt werden, der Abstand zwischen Cornea und Retina oder die Dicke der Linse. Das Biometer, das als solches bekannt ist, ist dazu ausgelegt, einen Messstrahl auf das Auge zu richten und anhand des reflektierten Lichts die erforderlichen Informationen zu ermitteln.

Es kann vorgesehen sein, dass in einem Okular und/oder auf einem Monitor des Operationsmikroskops, des Spaltlampenmikroskops bzw. des Biometers weitere Informationen für den Benutzer zur Verfügung gestellt werden. Die weiteren Informationen können dem optischen Bild und/oder dem Bild von den Kollagen-Strukturen überlagert werden. Vorzugsweise ist die Überlagerung derart, dass der Benutzer beides gleichzeitig sehen kann. Die weiteren Informationen können aus dem Bild oder aus der Strukturabbildung abgeleitete Information sein. Beispielsweise kann es sich um eine Cornea-Polarisationsachse oder davon abgeleitete Achsen und Punkte umfassen. Die Cornea-Polarisationsachse bezeichnet eine von den Kollagen-Strukturen definierte Achse. Die Kollagen-Strukturen spannen im Normalfall zwei Achsen auf, die im Wesentlichen senkrecht aufeinander stehen und von denen eine im Wesentlichen waagerecht und die andere im Wesentlichen senkrecht ausgerichtet ist. Durch die Einspiegelung einer Cornea-Polarisationsachse kann der Benutzer die Ausrichtung des Auges ablesen.

Eine weitere Anwendung des erfindungsgemäßen Verfahrens ist das so genannte Eye-Tracking. Bewegt sich das Auge während der Untersuchung, so verschiebt oder verdreht sich das mit dem Bildsensor aufgenommene Bild. Durch einen Vergleich mit einem zuvor aufgenommenen Bild ergibt sich ein Maß für die Verschiebung bzw. Verdrehung. Dies kann für eine entsprechende Nachführung genutzt werden. Beispielsweise kann das Operationsmikroskop, Spaltlampenmikroskope oder Biometer so nachgeführt werden, dass das Bild zentriert bleibt. Alternativ oder zusätzlich dazu kann eine Einspielung so nachgeführt werden, dass sie relativ zu dem Auge ihre Position behalt.

Die Erfindung betrifft außerdem ein Verfahren zum Vergleichen einer Augenstruktur mit einer Strukturabbildung. Bei dem Verfahren wird mit einem ersten Polarisator linear polarisiertes Messlicht in Richtung eines Auges gesendet, so dass linear polarisiertes Messlicht auf das Auge gerichtet wird und dass linear polarisiertes Messlicht auf das Auge gerichtet wird und dass linear polarisiertes Messlicht auf das Auge gerichtet wird und dass ein Anteil des Messlichts von dem Auge reflektiert wird. Im Strahlengang des reflektierten Messlichts wird ein zweiter Polarisator angeordnet, so dass die lineare Polarisationsrichtung des ersten Polarisators mit der linearen Polarisationsrichtung des zweiten Polarisators einen Winkel zwischen 75° und 105°, vorzugsweise einen Winkel zwischen 85° und 95°, weiter vorzugsweise einen Winkel von 90° einschließt. Es wird mittels eines Bildsensors ein Bild des durch den zweiten Polarisator hindurch getretenen Messlichts aufgenommen. Das Bild wird mittels eines Komparators mit einer Strukturabbildung verglichen. Das Bild und die Strukturabbildung enthalten Informationen über die Anordnung der Kollagen-Strukturen in der Cornea.

Es kann eine Mehrzahl von Einzelbildern des durch den zweiten Polarisator hindurchgetretenen Messlichts bei jeweils unterschiedlichen Ausrichtungen der Polarisation aufgenommen werden. Vorzugsweise wird bei jeder Ausrichtung der Polarisation der Winkel zwischen der linearen Polarisationsrichtung des ersten Polarisators und der linearen Polarisationsrichtung des zweiten Polarisators beibehalten. Die Einzelbilder können zu einem Gesamtbild überlagert werden und das Gesamtbild mit der Struktur-abbildung verglichen werden.

Die Strukturabbildung kann zu einem anderen Zeitpunkt an demselben Auge oder einem anderen Auge aufgenommen worden sein. Alter-nativ kann die Strukturabbildung eine Musterstruktur wiedergeben.

Wird die Strukturabbildung zu einem anderen Zeitpunkt an demselben Auge aufgenommen, so geschieht dies bei einer Ausführungsform des erfindungsgemäßen Verfahrens mit einem Biometer oder einem Spaltlampenmikroskop, das dazu ausgelegt ist, die erfindungsgemäße Messung durchzuführen und die Strukturabbildung zu speichern. Der Vergleich wird zu einem späteren Zeitpunkt durchgeführt, nachdem die erfindungsgemäße Messung ein weiteres Mal durchgeführt wurde und mit dem Bildsensor ein Bild aufgenommen wurde. Vorzugsweise wird die spätere Messung mit einer erfindungsgemäßen Anordnung durchgeführt, die ein Operationsmikroskop umfasst. Der Komparator kann in das Operationsmikroskop integriert sein.

Das Verfahren kann mit weiteren Merkmalen fortgebildet werden, die im Zusammenhang des erfindungsgemäßen Verfahrens beschrieben sind.

Die Erfindung wird nachfolgend unter Bezugnahme auf die beigefügten Zeichnungen anhand vorteilhafter Ausführungsform beispielhaft beschrieben. Es zeigen:
- Fig. 1:: ein Ausführungsbeispiel einer erfindungsgemäßen Anordnung;
- Fig. 2:: eine Ansicht von unten auf die Polarisatoren der Anordnung gemäß Fig. 1;
- Fig. 3:: ein Beispiel einer Strukturabbildung;
- Fig. 4:: ein Beispiel eines mit dem erfindungsgemäßen Bildsensor gewonnenen Bilds;
- Fig. 5:: eine weitere Ausführungsform einer erfindungsgemäßen Anordnung;
- Fig. 6:: eine Ansicht von unten auf die Polarisatoren der Anordnung gemäß Fig. 5;
- Fig. 7:: eine erfindungsgemäße Anordnung, umfassend ein Operationsmikroskop; und
- Fig. 8:: eine erfindungsgemäße Anordnung, umfassend einen Biometer.

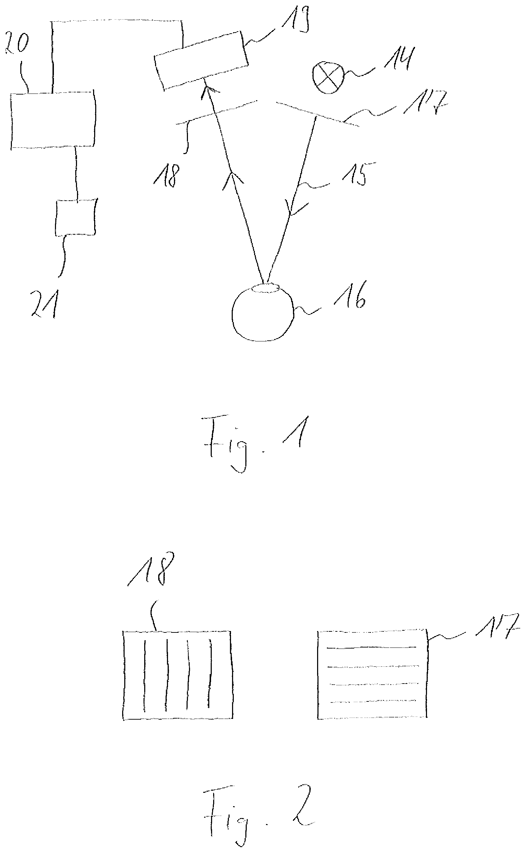

Eine erfindungsgemäße Anordnung in Fig. 1 umfasst eine Lichtquelle 14, die Messlicht 15 in Richtung eines Auges 16 sendet. Auf dem Weg von der Lichtquelle 14 zu dem Auge 16 tritt das Messlicht 15 durch einen ersten Polarisator 17 hindurch, der als linearer Polarisationsfilter ausgebildet ist. Das Messlicht 15, das auf dem Auge 16 auftrifft, ist folglich linear polarisiert.

Ein Teil des Messlichts 15 wird von dem Auge zurückgeworfen und trifft auf einen zweiten Polarisator 18, der ebenfalls als linearer Polarisationsfilter ausgebildet ist. Die Polarisationsrichtung des ersten Polarisators 17 schließt mit der Polarisationsrichtung des zweiten Polarisators 18 einen rechten Winkel ein. Dies ist in Fig. 2 gezeigt. Bei dieser Ausrichtung der Polarisatoren 17, 18 wird von dem Auge 16 direkt reflektiertes Messlicht 15 im Wesentlichen vollständig blockiert, weil der zweite Polarisator 18 gerade die Polarisationsrichtung vollständig sperrt, die von dem ersten Polarisator 17 hindurch gelassen wird.

Durch den zweiten Polarisator 18 hindurchtreten können lediglich solche Anteile des reflektierten Messlichts 15, deren Polarisationsrichtung sich zwischen dem ersten Polarisator 17 und dem zweiten Polarisator 18 verändert hat. Zu einer Veränderung der Polarisationsrichtung kommt es, wenn das Messlicht 15 auf eine Substanz trifft, die eine optische Aktivität aufweist. Durch eine Wechselwirkung mit der optisch aktiven Substanz ändert sich die Polarisationsrichtung des Messlichts 15, so dass der betreffende Anteil des reflektierten Messlichts 15 durch den zweiten Polarisator 18 hindurchtreten kann.

Eine optische Aktivität haben im Auge 16 die Kollagen-Strukturen der Cornea. Messlicht 15, das nach einer Wechselwirkung mit diesen Kollagen-Strukturen zurückgeworfen wird, tritt also durch den zweiten Polarisator 18 hindurch. Mit einem Bildsensor 19, der hinter dem zweiten Polarisator 18 angeordnet ist, kann folglich ein Bild dieses Anteils des Messlichts 15 aufgenommen werden. Ein Komparator 20 ist dazu ausgelegt, das Bild mit einer in einem Speicher 21 hinterlegten Strukturabbildung zu vergleichen.

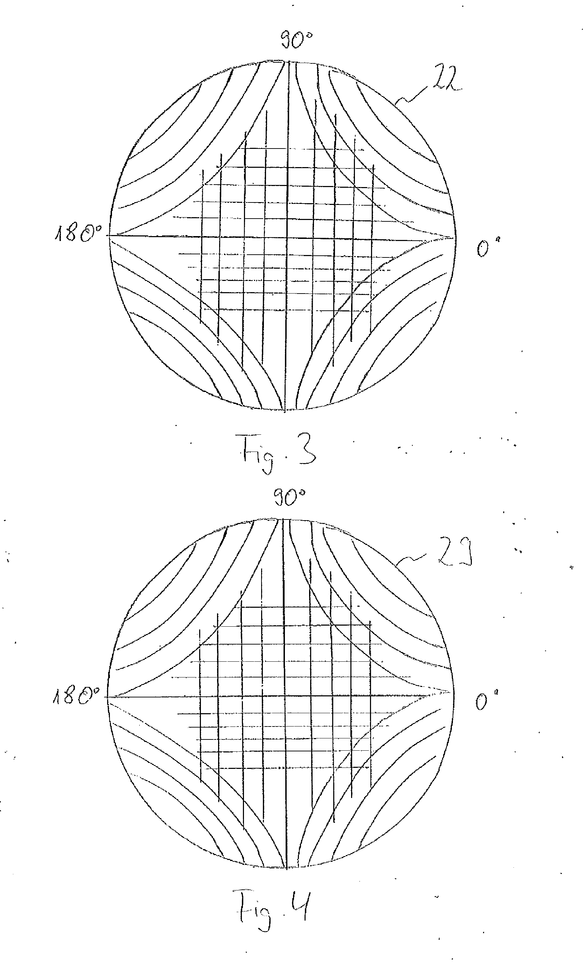

Ein Beispiel für eine Strukturabbildung 22 ist in Fig. 3 gezeigt. Die kreisrunde Umrandung entspricht der Fläche der Hornhaut des Auges 16. In der Hornhaut gibt es Kollagen-Strukturen, die sich bogenförmig in der Hornhaut erstrecken. Diese bogenförmigen Strukturen sind in der Strukturabbildung 22 angedeutet.

Die Fig. 4 zeigt ein mit dem Bildsensor 19 aufgenommenes Gesamtbild 29, das aus einer Mehrzahl von Einzelbildern überlagert ist. Der Komparator 20 vergleicht das Gesamtbild 29 mit der Strukturabbildung 22, indem er beide überlagert, und stellt fest, dass beide übereinstimmen. Da die Kollagen-Strukturen bei jedem Auge individuell verschieden sind, kann aus dieser Übereinstimmung gefolgert werden, dass das Gesamtbild 29 von demselben Auge stammt wie die Strukturabbildung 22. Der Komparator 20 kann dazu ausgelegt sein, ein Signal auszugeben, dass Übereinstimmung besteht. Bei einer chirurgischen Operation kann das Signal beispielsweise als Bestätigung dienen, dass die Behandlung am richtigen Auge vorgenommen wird.

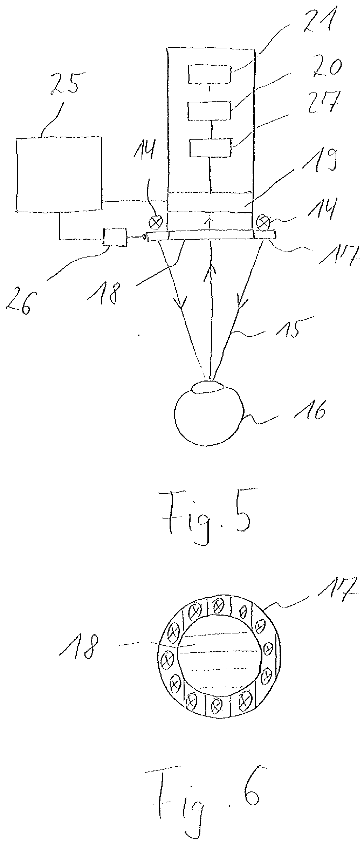

Bei der in Fig. 5 gezeigten erfindungsgemäßen Anordnung sind der erste Polarisator 17 und der zweite Polarisator 18 konzentrisch zueinander angeordnet. Der erste Polarisator 17 ist ringförmig und erstreckt sich um den zweiten Polarisator 18 herum, der scheibenförmig ist, siehe Fig. 6. Die Lichtquelle 14 für das Messlicht 15 wird von einer kreisförmigen Anordnung von Dioden gebildet, die hinter dem ersten Polarisator 17 angeordnet ist. Die lineare Polarisationsrichtung des ersten Polarisators 17 schließt mit der linearen Polarisationsrichtung des zweiten Polarisators 18 einen rechten Winkel ein. Dies ist in Fig. 6 mit den geraden Linien angedeutet. Bei dieser Anordnung der Lichtquelle 14 und der Polarisatoren 17, 18 kann mit dem Bildsensor ein Einzelbild aufgenommen werden.

Die Polarisatoren 17, 18 sind miteinander verbunden, so dass der rechte Winkel zwischen den beiden Polarisationsrichtungen fest eingestellt ist. Die Polarisatoren 17, 18 sind drehbar relativ zu dem Bildsensor 19 gelagert, so dass die Ausrichtung der Polarisation einstellbar ist. Ein Antrieb 26, der unter der Kontrolle einer Steuereinheit 25 steht, treibt die Drehbewegung der Polarisatoren 17, 18 an. In einer zweiten Winkelposition der Polarisatoren 17, 18 kann ein weiteres Einzelbild aufgenommen werden, das einen anderen Ausschnitt der Kollagen-Strukturen wiedergibt. Überlagert man die beiden Einzelbilder, so erhält man ein Gesamtbild 29, das mehr Informationen über die Kollagen-Strukturen enthält als jedes der Einzelbilder. Der Komparator 20 vergleicht das Gesamtbild 29 mit einer Strukturabbildung 22, die in dem Speicher 21 hinterlegt ist.

Die Steuereinheit 25 ist dazu ausgelegt, ausgehend von einem ersten Einzelbild die Polarisatoren 17, 18 in 2°-Schritten weiterzudrehen. Nach jedem Schritt sendet die Steuereinheit 24 ein Signal an den Bildsensor 19, so dass der Bildsensor 19 ein weiteres Einzelbild aufnimmt. Dieser Vorgang wird neunzig Mal wiederholt, so dass die Einzelbilder den Winkelbereich von 0° bis 180° abdecken. Mit einem Bildmodul 27 werden die neunzig Einzelbilder zu einem Gesamtbild überlagert. Das Gesamtbild 29 enthält eine umfassende Information über die Anordnung der Kollagen-Strukturen. Eine beispielhafte Darstellung eines Gesamtbilds 29 zeigt Fig. 4. Die bogenförmigen Kollagen-Strukturen sind ähnlich vollständig dargestellt wie in der Strukturabbildung 22.

In einem in den Figuren nicht dargestellten Beispiel ergibt der von dem Komparator 20 durchgeführte Vergleich zwischen dem Gesamtbild 29 und der Strukturabbildung 22, dass die Anordnung der Kollagen-Strukturen identisch ist. Allerdings ist bezogen auf einen gemeinsamen Bezugspunkt das Gesamtbild 29 relativ zu der Strukturabbildung 22 verschoben. Der Komparator 20 kann dies feststellen sowie die Richtung und den Abstand der Verschiebung ausgeben.

In einem weiteren Beispiel ist das Gesamtbild 29 gegenüber der Strukturabbildung 22 verdreht. Der Komparator 20 kann dies feststellen und einen Winkel ausgeben, um den das Gesamtbild 29 gedreht werden muss, um es zur Überdeckung mit der Strukturabbildung 22 zu bringen.

Bei einem weiteren nicht gezeigten Beispiel gibt das Gesamtbild 29 eine andere Kollagen-Struktur wieder als die Strukturabbildung 22. Es ist nicht möglich, die beiden Bilder zur Überdeckung zu bringen. Der Komparator 20 stellt dies fest und kann ein Warnsignal ausgeben, dass das Auge, auf das das Messlicht 15 gerichtet ist, ein anderes ist als das Auge, von dem die Strukturabbildung 22 stammt.

In einem weiteren Beispiel stimmt die Kollagen-Struktur des Gesamtbilds 29 zwar grundsätzlich mit der Strukturabbildung 22 überein. Allerdings zeigt das Gesamtbild 29 in einem Quadranten eine Veränderung, die darauf hindeuten kann, dass die Form der Hornhaut sich verändert hat seit dem Zeitpunkt, zu dem die Strukturabbildung 22 aufgenommen wurde. Der Komparator 20 stellt dies fest und kann ein Signal ausgeben, dass eine nähere Untersuchung erforderlich ist.

Schließlich ist es auch möglich, in das Gesamtbild 29 mit einer Musterstruktur zu vergleichen, die charakteristische Merkmale eines gesunden Auges wiedergibt. Abweichungen zwischen dem Gesamtbild 29 und der Musterstruktur können ein Hinweis auf beispielsweise einen Astigmatismus oder einen Keratokonus sein.

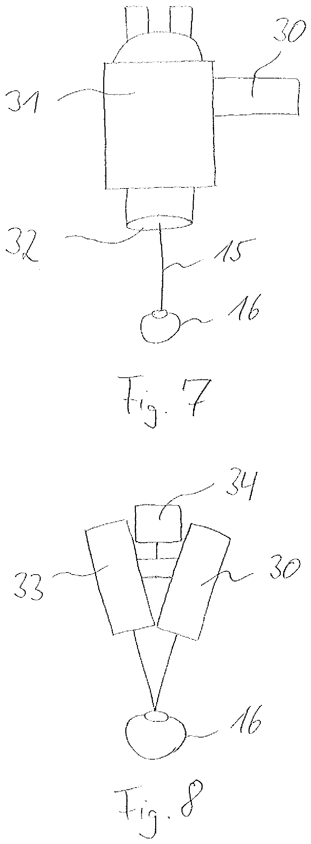

Die Fig. 7 zeigt ein Operationsmikroskop 31, das mit einer nur schematisch dargestellten Anordnung 30 gekoppelt ist. Das von der erfindungsgemäßen Anordnung 30 ausgehende Messlicht 15 wird dem Beobachtungsstrahlengang des Operationsmikroskops 31 koaxial überlagert und durch das Hauptobjektiv 32 des Operationsmikroskops 31 auf das Auge 16 geleitet. Das mit der Anordnung 30 gewonnene Bild 29 kann dem Beobachtungsstrahlengang des Operationsmikroskops 31 überlagert werden, so dass der Augenarzt das Bild 29 gleichzeitig mit dem optischen Bild des Auges 16 sehen kann. Möglich ist auch, nur eine Information über das Ergebnis des Vergleichs mit der Strukturabbildung 22 in das Operationsmikroskop einzublenden. Die Einblendung kann beispielsweise in einer Kennzahl oder in einer Warnung bestehen.

In Fig. 8 ist die erfindungsgemäße Anordnung 30 mit einem Biometer 33 gekoppelt. Die Anordnung 30 ermittelt ein Bild der Kollagen-Strukturen des Auges. Das Biometer 33 richtet einen Messstrahl auf das Auge 16, um das Auge zu vermessen. Die jeweiligen Informationen werden in einer gemeinsamen Steuereinheit 34 zusammengeführt.

Das erfindungsgemäße Verfahren kann so durchgeführt werden, dass in einem ersten Schritt mit dem Biometer 33 eine Strukturabbildung 22 aufgenommen wird. Die Strukturabbildung 22 wird in einem Speicher 21 abgelegt. Zu einem späteren Zeitpunkt wird mit dem Operationsmikroskop 31 eine weitere Messung durchgeführt. Der Komparator 20 des Operationsmikroskops vergleicht das aufgenommene Bild mit der zuvor von dem Biometer 33 gespeicherten Strukturabbildung 22.

## Patentansprüche

1. Anordnung zum Vergleichen einer Augenstruktur mit einer Strukturabbildung (22), umfassend folgende Merkmale:
a. einen Speicher (21) zum Speichern einer Strukturabbildung (22);
b. eine mit einem ersten linearen Polarisator (17) versehene Lichtquelle (14), um auf ein Auge (16) gerichtetes linear polarisiertes Messlicht (15) zu erzeugen;
c. einen Bildsensor (19) für von dem Auge (16) zurückgeworfene Anteile des Messlichts (15); und
d. einen zweiten Polarisator (18), der zwischen dem Auge (16) und dem Bildsensor (19) angeordnet ist, wobei die Polarisationsrichtung des ersten Polarisators (17) mit der Polarisationsrichtung des zweiten Polarisators (18) einen Winkel zwischen 75° und 105°, vorzugsweise einen Winkel zwischen 85° und 95°, weiter vorzugsweise einen Winkel von 90°, einschließt ; **dadurch gekennzeichnet, dass** die Anordnung zusätzlich umfasst
e. einen Komparator (20), der dazu ausgelegt ist, ein durch den Bildsensor (19) gewonnenes Bild (29) mit der Strukturabbildung (22) zu vergleichen; wobei das gewonnene Bild (29) und die Strukturabbildung Informationen über die Anordnung der Kollagen-Strukturen in der Cornea enthalten.

2. Anordnung nach Anspruch 1, **dadurch gekennzeichnet, dass** der erste Polarisator (17) und der zweite Polarisator (18) drehbar gelagert sind.

3. Anordnung nach Anspruch 2, **dadurch gekennzeichnet, dass** der erste Polarisator (17) und der zweite Polarisator (18) mit-einander gekoppelt sind.

4. Anordnung nach einem der Ansprüche 1 bis 3, **gekennzeichnet durch** ein Bildmodul (27), das dazu ausgelegt ist, eine Mehrzahl von Einzelbildern des Bildsensors (19) zu einem Gesamtbild (29) zu überlagern.

5. Anordnung nach einem der Ansprüche 1 bis 4, **gekennzeichnet durch** eine Steuereinheit (25), die dazu ausgelegt ist, den ersten Polarisator (17) und den zweiten Polarisator (18) in eine jeweils vorgegebene Polarisationsrichtung zu drehen und den Bildsensor (19) auszulösen.

6. Anordnung nach einem der Ansprüche 1 bis 5, **dadurch gekennzeichnet, dass** der Komparator (20) dazu ausgelegt ist, die Abweichung zwischen dem Bild (29) und der Strukturabbildung (22) automatisch auszuwerten.

7. Anordnung nach Anspruch 6, **dadurch gekennzeichnet, dass** der Komparator (20) dazu ausgelegt ist, einen die Abweichung repräsentierenden Kennwert zu ermitteln.

8. Anordnung nach Anspruch 6 oder 7, **dadurch gekennzeichnet, dass** der Komparator (20) dazu ausgelegt ist, ein Signal auszugeben, wenn die Abweichung größer ist als ein vorgegebener Schwellwert.

9. Anordnung nach einem der Ansprüche 1 bis 8, **dadurch gekennzeichnet, dass** sie ein Operationsmikroskop (31) und/oder ein Spaltlampenmikroskop für eine parallele optische Beobachtung des Auges (16) umfasst.

10. Anordnung nach einem der Ansprüche 1 bis 9, **dadurch gekennzeichnet, dass** sie ein Biometer (33) für eine parallele Vermessung des Auges (16) umfasst.

11. Anordnung nach Anspruch 9 oder 10, **dadurch gekennzeichnet, dass** in einem Okular und/oder auf einem Bildschirm eine weitere Information dargestellt ist, die dem Bild (29) und/oder einem optischen Bild des Auges (16) überlagert ist.

12. Verfahren zum Vergleichen einer Augenstruktur mit einer Strukturabbildung (22) mit folgenden Schritten:
a. Senden von mit einem ersten Polarisator (17) linear polarisiertem Messlicht (15) in Richtung eines Auges (16), so dass linear polarisiertes Messlicht auf das Auge gerichtet wird und dass ein Anteil des Messlichts (15) von dem Auge (16) reflektiert wird;
b. Anordnen eines zweiten Polarisators (18) im Strahlengang des reflektierten Messlichts (15), so dass die lineare Polarisationsrichtung des zweiten Polarisators (18) mit der Polarisationsrichtung des ersten Polarisators (17) einen Winkel zwischen 75° und 105°, vor-zugsweise einen Winkel zwischen 85° und 95°, weiter vorzugsweise einen Winkel von 90° einschließt; und
c. Aufnehmen eines Bilds (29) mittels eines Bildsensors (19) des durch den zweiten Polarisator (18) hindurchgetretenen Messlichts (15); **gekennzeichnet durch** den Schritt
d. Vergleichen des Bilds (29) mittels eines Komparators (20) mit einer Strukturabbildung (22), wobei das Bild (29) und die Strukturabbildung Informationen über die Anordnung der Kollagen-Strukturen in der Cornea enthalten.

13. Verfahren nach Anspruch 12, **dadurch gekennzeichnet, dass** eine Mehrzahl von Einzelbildern mit einer jeweils verschiedenen Ausrichtung der Polarisation von erstem Polarisator und zweitem Polarisator aufgenommenen wird.

14. Verfahren nach Anspruch 13 **dadurch gekennzeichnet, dass** die Mehrzahl von Einzelbildern zu einem Gesamtbild (29) überlagert wird und dass das Gesamtbild (29) mit der Strukturabbildung (22) verglichen wird.

## Claims

1. Assembly for comparing an eye structure with a structure image (22), including the following features:
a. a memory device (21) for storing a structure image (22) ;
b. a light source (14) equipped with a first linear polariser (17) in order to create a linear polarised measuring light (15) directed at an eye (16);
c. an image sensor (19) for parts of the measuring light (15) reflected from the eye (16);
d. a second polariser (18), which is arranged between the eye (16) and the image sensor (19), whereby the direction of polarisation of the first polariser (17) forms an angle of between 75° and 105°, preferably an angle of between 85° and 95°, more preferably an angle of 90° with the direction of polarisation of the second polariser (18); and
e. a comparator (20), in order to compare an image (29) obtained by the image sensor (19) with the structure image (22); whereby the image obtained (29) and the structure image contain information about the arrangement of the collagen structures in the cornea.

2. Assembly according to Claim 1, **characterised in that** the first polariser (17) and the second polariser (18) are mounted so as to be rotatable.

3. Assembly according to Claim 2, **characterised in that** the first polariser (17) and the second polariser (18) are coupled together.

4. Assembly according to one of Claims 1 to 3, **characterised by** an image module (27) which is designed to superimpose a number of individual images from the image sensor (19) to produce an overall image (29).

5. Assembly according to one of Claims 1 to 4, **characterised by** a control unit (25) which is designed to rotate the first polariser (17) and the second polariser (18) in a direction of polarisation specified in each case and to activate the image sensor (19) .

6. Assembly according to one of Claims 1 to 5, **characterised in that** the comparator (20) is designed to evaluate the deviation between the image (29) and the structure image (22) automatically.

7. Assembly according to Claim 6, **characterised in that** the comparator (20) is designed to determine a parameter which is representative of the deviation.

8. Assembly according to Claim 6 or 7, **characterised in that** the comparator (20) is designed to emit a signal if the deviation is greater than a given threshold value.

9. Assembly according to one of Claims 1 to 8, **characterised in that** it includes an operating microscope (31) and/or a slit lamp microscope for parallel optical observation of the eye (16).

10. Assembly according to one of Claims 1 to 9, **characterised in that** it includes a biometer (33) for parallel measurement of the eye (16) .

11. Assembly according to Claim 9 or 10, **characterised in that** additional information is presented in an eyepiece and/or on a screen, which is superimposed on the image (29) and/or an optical image of the eye (16).

12. Method for comparing an eye structure with a structure image (22) with the following stages:
a. Sending linear measuring light (15) polarised with a first polariser (17) in the direction of an eye (16), so that linear polarised measuring light is directed at the eye and so that part of the measuring light (15) is reflected by the eye (16);
b. Arranging a second polariser (18) in the beam path of the reflected measuring light (15), so that the direction of linear polarisation of the second polariser (18) forms an angle of between 75° and 105°, preferably an angle of between 85° and 95°, more preferably an angle of 90° with the direction of polarisation of the first polariser (17);
c. Taking an image (29) by means of an image sensor (19) of the measuring light (15) passing through the second polariser (18); and
d. By means of a comparator (20), comparing the image (29) with a structure image (22), whereby the image (29) and the structure image contain information about the arrangement of the collagen structures in the cornea.

13. Method according to Claim 12, **characterised in that** a number of individual images are taken with a different direction of polarisation of the first polariser and second polariser in each case.

14. Method according to Claim 13 **characterised in that** the number of individual images are superimposed to produce an overall image (29) and that the overall image (29) is compared with the structure image (22).

## Revendications

1. Ensemble pour comparer une structure d'œil à une représentation de structure (22), comprenant des caractéristiques suivantes :
a. une mémoire (21) pour mémoriser une représentation de structure (22) ;
b. une source de lumière (14) pourvue d'un premier polariseur linéaire (17) pour produire de la lumière de mesure (15) polarisée linéairement, dirigée sur un œil (16) ;
c. un capteur d'image (19) pour des fractions de la lumière de mesure (15) réfléchie par l'œil (16) ; et
d. un deuxième polariseur (18) qui est disposé entre l'œil (16) et le capteur d'image (19), dans lequel la direction de polarisation du premier polariseur (17) forme avec la direction de polarisation du deuxième polariseur (18) un angle entre 75° et 105°, de préférence un angle entre 85° et 95°, de manière davantage préférée un angle de 90° ; **caractérisé en ce que** l'ensemble comprend en supplément
e. un comparateur (20) qui est conçu pour comparer une image (29) obtenue par le capteur d'image (19) à la représentation de structure (22) ; dans lequel l'image (29) obtenue et la représentation de structure contiennent des informations sur l'agencement des structures de collagène dans la cornée.

2. Ensemble selon la revendication 1, **caractérisé en ce que** le premier polariseur (17) et le deuxième polariseur (18) sont montés de manière à pouvoir tourner.

3. Ensemble selon la revendication 2, **caractérisé en ce que** le premier polariseur (17) et le deuxième polariseur (18) sont couplés l'un à l'autre.

4. Ensemble selon l'une quelconque des revendications 1 à 3, **caractérisé par** un module d'image (27) qui est conçu pour superposer une multitude d'images individuelles du capteur d'image (19) en une image globale (29).

5. Ensemble selon l'une quelconque des revendications 1 à 4, **caractérisé par** une unité de commande (25) qui est conçue pour faire tourner le premier polariseur (17) et le deuxième polariseur (18) dans une direction de polarisation respectivement prédéfinie et pour déclencher le capteur d'image (19).

6. Ensemble selon l'une quelconque des revendications 1 à 5, **caractérisé en ce que** le comparateur (20) est conçu pour évaluer automatiquement l'écart entre l'image (29) et la représentation de structure (22).

7. Ensemble selon la revendication 6, **caractérisé en ce que** le comparateur (20) est conçu pour déterminer une valeur caractéristique représentant l'écart.

8. Ensemble selon la revendication 6 ou 7, **caractérisé en ce que** le comparateur (20) est conçu pour émettre un signal quand l'écart est supérieur à une valeur de seuil prédéfinie.

9. Ensemble selon l'une quelconque des revendications 1 à 8, **caractérisé en ce qu'**il comprend un microscope chirurgical (31) et/ou un microscope à lampe à fente pour une observation optique parallèle de l'œil (16).

10. Ensemble selon l'une quelconque des revendications 1 à 9, **caractérisé en ce qu'**il comprend un biomètre (33) pour une mesure parallèle de l'œil (16).

11. Ensemble selon la revendication 9 ou 10, **caractérisé en ce qu'**est représentée dans un oculaire et/ou sur un écran une autre information, qui est superposée à l'image (29) et/ou à une autre image optique de l'œil (16).

12. Procédé pour comparer une structure d'œil à une représentation de structure (22), avec des étapes suivantes :
a. l'envoi de lumière de mesure (15) polarisée linéairement avec un premier polariseur (17) en direction d'un œil (16) de sorte que de la lumière de mesure polarisée linéairement est dirigée sur l'œil et qu'une fraction de la lumière de mesure (15) est réfléchie par l'œil (16) ;
b. l'agencement d'un deuxième polariseur (18) sur le chemin optique de la lumière de mesure (15) réfléchie de telle sorte que la direction de polarisation linéaire du deuxième polariseur (18) forme avec la direction de polarisation du premier polariseur (17) un angle entre 75° et 105°, de préférence un angle entre 85° et 95°, de manière davantage préférée un angle de 90° ; et
c. l'enregistrement d'une image (29) au moyen d'un capteur d'image (19) de la lumière de mesure (15) ayant traversé le deuxième polariseur (18) ;
d. la comparaison de l'image (29) au moyen d'un comparateur (20) à une représentation de structure (22), dans lequel l'image (29) et la représentation de structure contiennent des informations sur l'agencement des structures de collagène dans la cornée.

13. Procédé selon la revendication 12, **caractérisé en ce qu'**une multitude d'images individuelles est enregistrée avec une orientation respectivement différente de la polarisation du premier polariseur et du deuxième polariseur.

14. Procédé selon la revendication 13, **caractérisé en ce que** la multitude d'images individuelles est superposée en une image globale (29), et que l'image globale (29) est comparée à la représentation de structure (22).
